# EUROPEAN PATENT APPLICATION

(11) **EP 4 397 318 A1**
(43) Date of publication of application: **10.07.2024**
(21) Application number: 21955905.1
(22) Date of filing: 30.08.2021
(51) Int. Cl.: A61K 47/64, A61K 39/145, A61K 39/187, A61K 39/215, A61P 31/12

(54) **IMMUNOSTIMULATORY FORMULATION, AND COSMETIC, FOOD, FEED ADDITIVE, AND QUASI-DRUG CONTAINING SAID IMMUNOSTIMULATORY FORMULATION**

(71) Applicant: Japan Eco-Science Co. Ltd., Chiba-shi, Chiba, 263-8522 (JP); Miroku Co. Ltd., Oita 873-0021 (JP)
(72) Inventor: MIYAMOTO Hirokuni, Chiba-shi, Chiba 263-8522 (JP); MIYAMOTO Hisashi, Kitsuki-shi, Oita 873-0021 (JP)
(74) Representative: Hauck Patentanwaltspartnerschaft mbB
(86) International application number: PCT/JP2021/031769
(87) International publication number: WO 2023/032008

(57) **Abstract**

Without being limited to an epitope of a conventional antigen, it is possible to perform relatively simple formulation for activating a mucosal immune system and inducing highly diverse immunoglobulins by a non-invasive method. In a conventional vaccine, it is difficult to select an epitope antigen, and it takes time to approve the vaccine; therefore, it is assumed that it may be difficult to obtain an effect after approval for a pathogen target such as a virus that is likely to be mutated. In addition, since vaccination is not basically administered by the non-invasive method, suitable medical practice in a medical institution has been essential. Provided is an immunostimulatory preparation which is a protein obtained by fusing a bacteria-derived heat shock protein (HSP: heat shock protein) and a viral peptide antigen, and enables induction of a highly diverse immunoglobulin by simultaneously stimulating nasal, respiratory, and oral administration. Provided are a feed, a feed additive, and an environmental symbiotic preparation for livestock, or a cosmetic, a food, and a quasi-pharmaceutical product for humans, which enable symbiosis with an environmental microorganism containing the immunostimulatory preparation. Provided are a preparation, a cosmetic for humans, a food, and a quasi-pharmaceutical product that enable symbiosis with environmental microorganisms utilizing livestock-derived IgA, IgG, and IgY among the immunoglobulins.

## Description

### TECHNICAL FIELD

The present invention relates to an environmental symbiotic immunostimulatory preparation for inducing a diverse immunoglobulin to be administered nasally, transtracheally or orally, and uses thereof.

### BACKGROUND ART

In recent years, techniques in life science have progressed at various angles, an omics analysis technique for evaluating complex microorganisms and compositions of complex metabolites has been established, and understanding of various life phenomena has been advanced by machine learning, utilization of artificial intelligence, and the like. On the other hand, countermeasures against various unknown infectious diseases which are pointed out to be associated with various problems in an ecosystem caused by environmental destruction and environmental destruction are urgent problems not only for humans but also for livestock, and comprehensive measures are desired.

Although vaccines are used as a method for preventing infectious diseases, in general, live vaccines and vaccines in which the functions of microorganisms are inactivated are mainly used. In recent years, new techniques such as RNA vaccines and DNA vaccines utilizing genetic information of microorganisms, and plant-derived vaccines expressed in plants utilizing genetic information have also been developed (Non Patent Literatures 1, 2, 3, and 4). These vaccines are primarily aimed at selectively inducing IgG that produces a specific effect against a target pathogenic microorganism.

On the other hand, there is a nasal vaccine as a vaccine intended to induce secretory IgA having an effect on the pathogenic microorganism although the titer is weak, and some of the vaccines are commercially available (Non Patent Literatures 1 and 5).

In the oral vaccine, a system that utilizes M cells in Peyer's patches of intestinal lymphatic tissue has been devised (Patent Literature 1 and Non Patent Literature 6). It is known that the M cells express GP-2 and a cell surface molecule called PrP, take up bacteria, and serve as an entrance of an immune response via dendritic cells (Non Patent Literatures 7 and 8). Thereafter, several studies have been conducted, and it is been suggested that bacteria-derived Heat shock protein 60 (HSP 60) plays an important role in binding to the surface molecule (Non Patent Literatures 7, 8, and 9). In addition, it is suggested that a similar system of antigen uptake by the M cells is also observed in nasal mucous membranes and respiratory mucous membranes (Non Patent Literatures 10 and 11).

In the activation of an immune system in the respiratory tract, it is known that bacteria-derived Heat shock protein 65 (HSP 65), particularly HSP 65 derived from Mycobacterium leprae has an immunostimulatory effect (Non Patent Literatures 12 and 13). On the other hand, attempts have also been made to develop vaccines against Mycobacterium tuberculosis utilizing these effects (Non Patent Literatures 14 and 15). As described above, in a mucosal system, the possibility that HSP 60 and HSP 65 regulate biophylaxis by binding to cells or having adjuvant activity and the like is suggested.

On the other hand, when looking at a symbiotic relationship between animals and bacteria, in recent years, a relationship between an intestinal bacterial flora and a physiological function/disease state of a host has been rapidly clarified. For example, it is known that while 1,000 or more kinds of more than 40 trillion intestinal bacteria inhabit in a human intestinal tract, a complex intestinal ecosystem is formed, and they interact with host tissues to contribute to health maintenance. Therefore, when a balance of these intestinal ecosystems is disturbed, so-called dysbiosis leads to an onset of various diseases (Non Patent Literatures 16 and 17), and therefore, the need for probiotics that control inside of intestine and prebiotics has been recognized.

Incidentally, the present inventors have succeeded in the development of probiotics that affect the living body of animals by utilizing a thermophilic Bacillus bacterial group, which is one of extreme environmental microorganisms that are difficult to grow in a normal temperature region, and in particular, as livestock such as chickens, pigs, and cattle, fish, and rodents as model animals, the present inventors suggest a possibility that the intestinal bacterial flora of mice and rats can be controlled, and at that time, the living body can be favorably influenced (Patent Literatures 2 and 3 and Non Patent Literature 18).

An undeveloped region is the control of intestinal viruses. In general, when looking at a symbiotic relationship between animals and viruses, although it has been suggested that phage plays an important role in a biophylaxis system in the intestinal tract (Non Patent Literature 19), it cannot be said that the research on phages is necessarily more advanced than the research on intestinal bacteria.

However, for example, the spread of porcine epidemic diarrhea virus (Alphacoronavirus) as a virus capable of infecting the mucosal immune system of animals is problematic in the swine industry. The virus has a property that it is not fatal in a mother pig or a fattening pig, and is highly fatal when a young pig is infected (Non Patent Literature 20). Although an infection mode is different from that of SARS-cov and SARS-cov-2 which are Betacoronavirus in the same coronavirus (Non Patent Literature 21), they have similar properties in that they have Spike protein (Non Patent Literature 22). However, in development research on these vaccines, independent development has progressed in the medical field and the livestock field, and has not progressed in consideration of similar virus characteristics. On the other hand, in 1796, Edward Jenner who was a precursor of a basic concept of vaccine showed that an onset of smallpox was suppressed by utilizing the pus of cowpox infecting cattle (Non Patent Literature 23). Later, it has been shown that the DNA sequences of the cowpox virus and the smallpox virus are very similar (Non Patent Literature 24). That is, the preventive effect is confirmed using another virus similar to and different from an actual pathogenic virus itself. In recent years, a mechanism that exerts an effect on an unintended disease is called an off-target effect (although it is not an original target therapeutic effect, it is also effective against other diseases), and this is exactly the case. From a background as a starting point of such vaccine development, there are many points that a current vaccine development deviates.

### CITATION LIST

### PATENT LITERATURE

Patent Literature 1: Japanese Patent Application No. 2006-331950
Patent Literature 2: Japanese Patent No. 5578375
Patent Literature 3: Japanese Patent No. 5041228

### NON PATENT LITERATURE

Non Patent Literature 1: 22nd Meeting of the Health Sciences Council, Subcommittee on Immunization and Vaccines, Subcommittee on Research and Development and Production and Distribution. December 25, 2019. "Research and Development of Vaccines". https://www.mhlw.go.jp/content/10906000/000580437.pdf
Non Patent Literature 2: DNA vaccine. https://ja.wikipedia.org/wiki/ DNA vaccine
Non Patent Literature 3: RNA vaccine. https://www.t.utokyo.ac.jp/shared/press/data/setnws_201710121450382322891478_608 693.pdf
Non Patent Literature 4: Plant-derived vaccine. https://www.mt-pharma.co.jp/ir/meeting/pdf/presen170927_M.pdf
Non Patent Literature 5: Nasal vaccine. https://www.amed.go.jp/news/release _20190104.html
Non Patent Literature 6: Oral vaccine. https://shingi.jst.go.jp/past_abst/abst/p/14/1407/riken2-5.pdf
Non Patent Literature 7: Hiroshi Ohno., Biology of M Cells, A Unique Subset of Intestinal Epithelial Cells. Biochemistry, VOL. 83, No. 1, pp. 13-2, 201. https://www.jbsoc.or.jp/seika/wp-content/uploads/2013/05/83-01-03.pdf
Non Patent Literature 8: Koji Hase., Biological Significance of M Cells in Gut Mucosal Immunity. Journal of Intestinal Microbiology. 26:11-17,2012. https://www.jstage.jst.go.jp/article/jim/26/1/26_1_11/_pdf
Non Patent Literature 9: Gaku Nakato, Koji Hase, Michio Suzuki, Masanobu Kimura, Manabu Ato, Misaho Hanazato, Minoru Tobiume, Motohiro Horiuchi, Ryuichiro Atarashi, Noriyuki Nishida, Masahisa Watarai, Koichi Imaoka and Hiroshi Ohno., Cutting Edge: Brucella abortus Exploits a Cellular Prion Protein on Intestinal M Cells as an Invasive Receptor. J Immunol 2012; 189:1540-1544; Prepublished online 6 July 2012; doi: 10.4049/jimmunol.1103332
Non Patent Literature 10: Shunsuke Kimura, Mami Mutoh, Meri Hisamoto, Hikaru Saito, Shun Takahashi, Takanori Asakura, Makoto Ishii, Yutaka Nakamura, Junichiro Iida, Koji Hase, Toshihiko Iwanaga., Airway M cells arise in the lower airway due to RANKL signaling and reside in the bronchiolar epithelium associated with iBALT in murine models of respiratory disease. Frontiers in Immunology (2019) DOI: 10.3389/fimmu.2019.01323
Non Patent Literature 11: Tetsuo Himi, Minoru Go, Kenichi Takano, Makoto Kurose, Junichi Koizumi, et al., Mucosal Immune Barrier and Antigen Presenting System in Nasal Epithelial Cells of Allergic Rhinitis. Clinical Immunology and Allergology 47: 427-433, 2007.
Non Patent Literature 12: Yeong-Ho Rha, Christian Taube, Angela Haczku, Anthony Joetham, Katsuyuki Takeda, Catherine Duez, Marvin Siegel, M. Kemal Aydintug, Willi K. Born, Azzeddine Dakhama and Erwin W. Gelfand., Effect of Microbial Heat Shock Proteins on Airway Inflammation and Hyperresponsiveness. Immunol 2002; 169:5300-5307; doi: 10.4049/jimmunol.169.9.5300
Non Patent Literature 13: Yoo Seob Shin, Katsuyuki Takeda, Yoshiki Shiraishi, Yi Yeong Jeong, Joanne Domenico, Yi Jia, Junyan Han, Ralf Spallek, Mahavir Singh, Joseph J. Lucas, and Erwin W., Gelfand.Microbial Heat Shock Protein 65 Attenuates Airway Hyperresponsiveness and Inflammation by Modulating the Function of Dendritic Cells. Immunol. 2012 October 1; 189(7): 3404-3410. doi:10.4049/jimmunol.1201138
Non Patent Literature 14: Masaji Okada., The Development of Novel Vaccine for Tuberculosis. Jpn. J. Clin. Immunol., 31 (5) 356-368 (2008). https://www.jstage.jst.go.jp/article/jsci/31/5/31_5_356/_pdf
Non Patent Literature 15: Pryscilla Fanini Wowk, Luis Henrique Franco, Denise Morais da Fonseca, Marina Oliveira Paula, Elcio dos Santos Oliveira Vianna, Ana Paula Wendling, Valeria Maria Augusto, Silvana Maria Eloi-Santos, Andrea Teixeira-Carvalho, Flavia Dias Coelho Silva, Solange Alves Vinhas, Olindo Assis Martins-Filho, Moises Palaci, Celio Lopes Silva & Vania Luiza Deperon Bonato., Mycobacterial Hsp65 antigen upregulates the cellular immune response of healthy individuals compared with tuberculosis patients. HUMAN VACCINES & IMMUNOTHERAPEUTICS 2017, VOL. 13, No. 5, 1040-1050
Non Patent Literature 16: Claesson, Marcus J., et al., "Gut microbiota composition correlates with diet and health in the elderly. "Nature 488.7410 (2012): 178-184.
Non Patent Literature 17: Le Chatelier E, Nielsen T, Qin J, Prifti E, Hildebrand F, Falony G, Almeida M, Arumugam M, Batto JM, Kennedy S, Leonard P, Li J, Burgdorf K, Grarup N, Jorgensen T, Brandslund I, Nielsen HB, Juncker AS, Bertalan M, Levenez F, Pons N, Rasmussen S, Sunagawa S, Tap J, Tims S, Zoetendal EG, Brunak S, Clement K, Dore J, Kleerebezem M, Kristiansen K, Renault P, Sicheritz-Ponten T, de Vos WM, Zucker JD, Raes J, Hansen T; MetaHIT consortium, Bork P, Wang J, Ehrlich SD, Pedersen O., Richness of human gut microbiome correlates with metabolic markers. Nature 500:541-549. (2013)
Non Patent Literature 18: Miyamoto H, Seta M, Horiuchi S, Iwasawa Y, Naito T, Nishida A, Miyamoto H, Matsushita T, Itoh K, Kodama H., (2013) Potential probiotic thermophiles isolated from mice after compost ingestion. Journal of Applied Microbiology,114(4): 1147-1157
Non Patent Literature 19: Jeremy J. Barra,1, Rita Auroa, Mike Furlana, Katrine L. Whitesona, Marcella L. Erbb, Joe Poglianob, Aleksandr Stotlanda,Roland Wolkowicza, Andrew S. Cuttinga, Kelly S. Dorana, Peter Salamonc, Merry Youled, and Forest Rohwer., (2013) Bacteriophage adhering to mucus provide a non-host-derived immunity. Proc. Natl. Acad. Sci. vol.110, no.26, 10771-10776
Non Patent Literature 20: Hirokuni Miyamoto. "Chapter 22: Environmental Microorganisms and Animals". Hiroshi Ohno (ed.)., "Symbiotic Microorganisms". Chemistry Doujin (Dojin Bioscience Series No.27), p.247-256 (2016)
Non Patent Literature 21: Kristian G. Andersen, Andrew Rambaut, W. Ian Lipkin, Edward C. Holmes and Robert F. Garry., (2020) The proximal origin of SARS-CoV-2.Nature Mdicine vol.26,450-455 https://www.nature.com/articles/s41591-020-0820-9
Non Patent Literature 22: Xi-Mei Lei, Yong-Le Yang, Yong-Qiang He, Lei Peng, Pengwei Zhao, Shu-Ya Xu, Hongwei Cao, Pengfei Fang, Wenying Qiu, Pan Qin, Bin Wang, Yao-Wei Huang., (2019) Specific recombinant proteins of porcine epidemic diarrhea virus are immunogenic, revealing their potential use as diagnostic markers. Veterinary Microbiology.236,108387
Non Patent Literature 23: Akira Kajita., "History of Medicine". Kodansha
Non Patent Literature 24: Damaso, C., Revisiting Jenner's mysteries, the role of the Beaugency lumph in the evolutionary path of ancient smallpox vaccines. Lancet Infect. Dis., August 18, 2017
Non Patent Literature 25: Shinya Mizuno. Eriko Oosaki., "Literature review on the susceptibility of domestic animals toward SARS-CoV-2". January 2021. VOL. 5(1) p.94-97
Non Patent Literature 26: Shi J, et al., (2020) Science 368: 1016-1020
Non Patent Literature 27: Schlottau K, et al., (2020) Lancet Microbe 1: e218-e225
Non Patent Literature 28: Vergara Alert J. et al., (2020) Transbound Emerg Dis
Non Patent Literature 29: Ulrich L., et al., (2020) Emerg Infect Dis 26:2979-2981

### TECHNICAL PROBLEMS

In general, vaccine development is progressed by targeting a target pathogen itself or an epitope molecule characteristic of the pathogen. In addition, there are many technical restrictions on selection of epitopes, such as avoiding complicated structures such as an alpha helix and a beta sheet, and selecting a hydrophilic region. Therefore, although it is known that an infectious disease common to livestock and humans (including pet animals) exists, vaccine development considering a target infectious pathogen and a universal gene sequence common between the pathogen and a pathogen genetically related to the pathogen has hardly been performed.

As described above, in conventional vaccine development, since characteristics of an antigen (epitope region) to be a vaccine are localized in a specific virus, there is a high possibility of being useful for raising the titer of an antibody; however, it is not necessarily efficient when viewed from the viewpoint of countermeasures against infectious diseases common to humans and animals and symbiosis of the entire ecosystem.

For pathogens that are likely to undergo genetic mutation, such as RNA viruses, a conventional vaccine development technique has a weak point, and even if a vaccine is developed, it is assumed that the vaccine may not be effective against the mutated virus at the stage of approval.

In addition, in order to enhance the function of a vaccine, the immune system of an original host needs to be healthy; however, at present, research and development in view of a synergistic effect with a drug, a nutrient, a supplement, or the like for eliciting the effect of the vaccine have not been progressed.

### SOLUTION TO PROBLEMS

Whereas the manufacturing procedure of a conventional vaccine in modern times focuses on specificity of target pathogenic bacteria, a characteristic of a procedure using the cowpox pus of Edward Jenner, who gave rise to the concept of the vaccine itself, as described above, is to take measures against the smallpox virus from a different cowpox virus, which is similar in DNA sequence also from a later detailed analysis. That is, as a result, there is a direction of vaccine development in consideration of a genetic similarity of the virus itself, and there is space for focusing on these differences again. Interestingly, as in the literature (25 to 29), it has been found that in livestock, although there is a possibility of infection, the virus does not proliferate and is not infectious. It is currently known that livestock are infected with not a betacoronavirus like SARS-Cov-2 that is problematic, but a closely related alphacoronavirus. Although porcine epidemic diarrhea (PED) has become a problem worldwide in livestock industry associations, the PED virus belongs to alphacoronavirus. While such infection spread has been recognized worldwide, there may be a possibility that livestock having acquired immunity in advance may have acquired infection resistance by acquiring humoral immunity. This hypothesis is an off-target effect that does not contradict the viewpoint of Literature 1, and is expected to be one of cross immunity.

Thus, in the development of the present preparation, targets are narrowed down to a genetically common sequence with relatively closely related viruses, and these are used as epitope antigens, and are fused with a bacteria-derived HSP even if not necessarily in a hydrophilic region, thereby inducing stimulation of the immune system via dendritic cells utilizing characteristics of the M cells of mucosal tissue capable of taking up the bacteria-derived HSP.

In addition, the whole of the mucosal system is moved by nasal, respiratory, or oral administration, and soundness of the intestinal bacterial flora is improved together with probiotics and prebiotics, thereby enhancing the whole biological defense function.

The world famous hygiene hypothesis (Strachan DP. Hayfever, hygiene, and householdsize. BMJ 299: 12591260, 1989) is a hypothesis indicating that a difference in exposure of environmental factors in early childhood causes a difference in incidence of subsequent allergies and that the non-hygienic environment reduces an onset of the subsequent allergies; however, this hypothesis is now widely recognized, many data supporting this hypothesis are accumulated, and a relationship with other diseases in relation to the formation of the intestinal bacterial flora is also pointed out. That is, this suggests that as animals live, it is important to construct an environment in which the animals can live together with an environmental microbial community.

On the other hand, since the present preparation is targeted at molecules common to closely related microorganisms, without sticking to microorganisms that have already entered the living sphere, it is possible to stimulate the mucosal immune system to artificially establish a symbiotic relationship with unknown environmental microorganisms. This may also provide protection against unknown viruses. Therefore, when the whole animal is considered, the present preparation is an unconventional preparation for constructing a symbiotic relationship with environmental microorganisms, and in the case of livestock, it is assumed as a better application to be put in a functional feed additive, an unconventional type of environmental purifier, cosmetics, daily necessities such as aromatherapy, foods, and the like for the purpose of replacement with human.

Thus, provided is an immunostimulatory preparation characterized by having a fusion protein in which heat shock protein (HSP) or the amino acid-modified HSP is bound to a peptide epitope sequence, and solubility is improved as compared with the condition of the peptide epitope alone.

In addition, the immunostimulatory preparation is characterized by including a fusion protein in which a plurality of types of units including different base sequences are bound before and after the peptide epitope, and then the HSP or the amino acid-modified HSP is bound.

In addition, the immunostimulatory preparation is characterized in that after the HSP or the amino acid-modified HSP is taken up by M cells of mucous membranes in a nasal cavity, a respiratory tract, and an intestinal tract and the peptide epitope is presented as an antigen, and then cross immunity against a microorganism which is likely to be mutated is induced depending on the sequence of the peptide epitope and properties of different base sequences before and after the peptide epitope.

In addition, the immunostimulatory preparation is characterized by including, as a unit including different base sequences before and after the peptide epitope, an epitope on a surface of a virus common to or similar to various strains of a coronavirus that is likely to be mutated and a sequence before and after the epitope, the sequence being at least an amino acid sequence of any one of SEQ ID NO: 28 common to an alphacoronavirus and a betacoronavirus, and SEQ ID NO: 29 or SEQ ID NO: 30 similar between the alphacoronavirus and the betacoronavirus.

The microorganism which is likely to be mutated is a pestivirus, and the peptide epitope includes at least any one amino acid sequence of SEQ ID NOs: 31 to 37.

The microorganism which is likely to be mutated is an influenza virus, and the peptide epitope includes at least any one amino acid sequence of SEQ ID NOs: 38 to 40.

### ADVANTAGEOUS EFFECTS OF INVENTION

The present invention is a vaccine-like immunostimulant for forming an immunoglobulin having a low titer and a wide binding capacity. When a pandemic problem due to an unknown virus occurs, it is necessary to develop a vaccine as strong as possible; however, the authentication of a conventional vaccine requires a period of time in units of years. Therefore, the preparation is intended to reduce the risk of developing an infection and prevent an increase in the number of patients who become serious while having a low titer in its previous stage or in a stage where the development has failed.

In particular, although secretory IgA that plays a major role in mucosal immunity is induced, for example, since RNA viruses are easily mutated, it is a point that a preventive effect can be exerted to some extent even if the titer is low by utilizing IgA having a relatively wide binding capacity even when mutated, and the conventional problem is solved.

In addition, the present invention is expected to be able to efficiently protect against a virus that is likely to be mutated if there is a technique that is highly safe, is resistant to mutation, and has high versatility. It is expected that the effect of the immunostimulant is further enhanced by using the immunostimulant in combination with a conventional vaccine.

### BRIEF DESCRIPTION OF THE DRAWINGS

Fig. 1 shows production of crossed/diverse immunoglobulin IgY by hybrid antigen and use thereof.

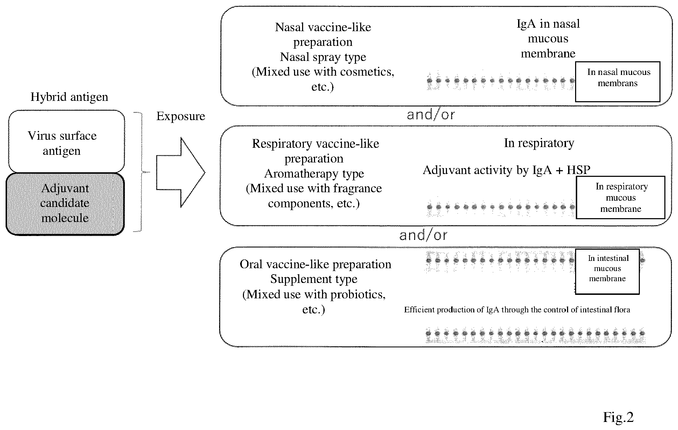

Fig. 2 shows an image of use of a crossed/diverse IgA-induced vaccine different from a conventional vaccine.
Fig. 3 shows an image of antigen presentation in a mucosal tissue exposed to a hybrid antigen.

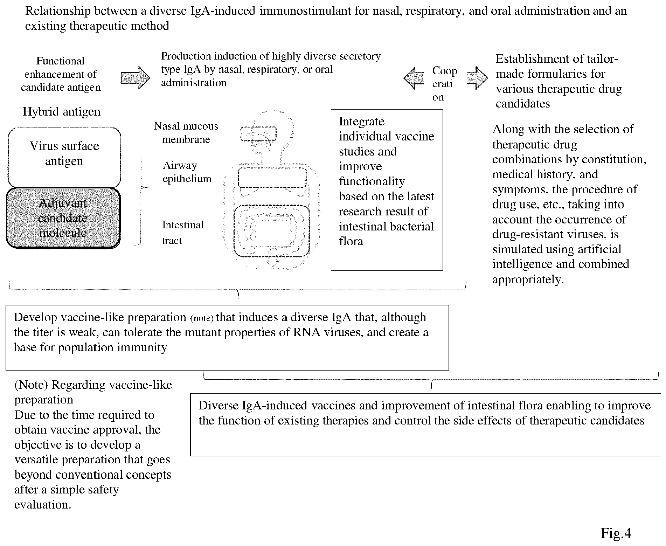

Fig. 4 shows a relationship between a crossed/diverse IgA-induced immunostimulant for nasal, respiratory, and oral administration and an existing therapeutic method, and a future view.
Fig. 5 shows a comparative genome analysis of spike proteins of an alphacoronavirus and a betacoronavirus (sequence region I).
Fig. 6 shows the comparative genome analysis of the spike proteins of the alphacoronavirus and the betacoronavirus (sequence region II).
Fig. 7 shows an epitope target I of the spike proteins of the alphacoronavirus and the betacoronavirus.
Fig. 8 shows the epitope target II of the spike proteins of the alphacoronavirus and the betacoronavirus.
Fig. 9 shows a case example of a hybrid antigen.
Fig. 10 shows common/similar sequence regions of the alphacoronavirus and the betacoronavirus.
Fig. 11 shows the common/similar sequence regions of the alphacoronavirus and the betacoronavirus.
Fig. 12 shows synthetic protein amino acid sequence information.
Fig. 13 is a design application for other viruses.

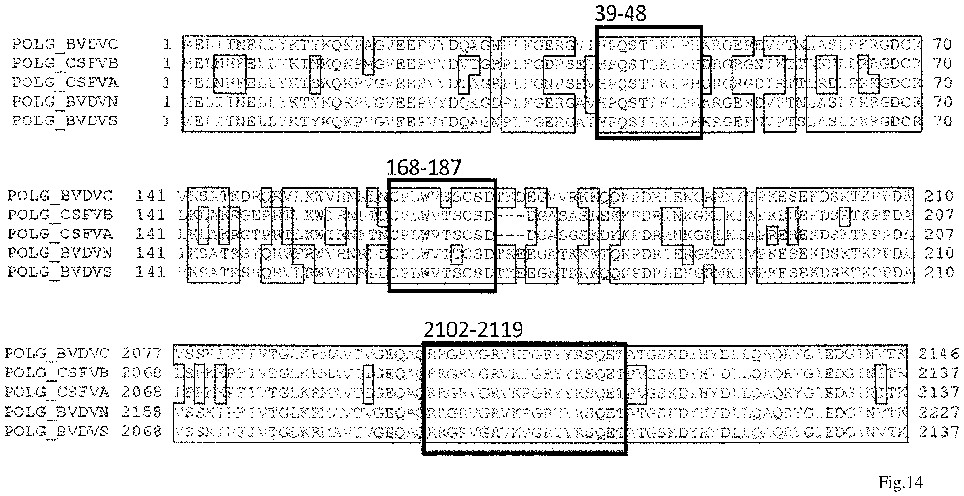

Fig. 14 shows common/similar sequence information of swine fever virus and related viruses.

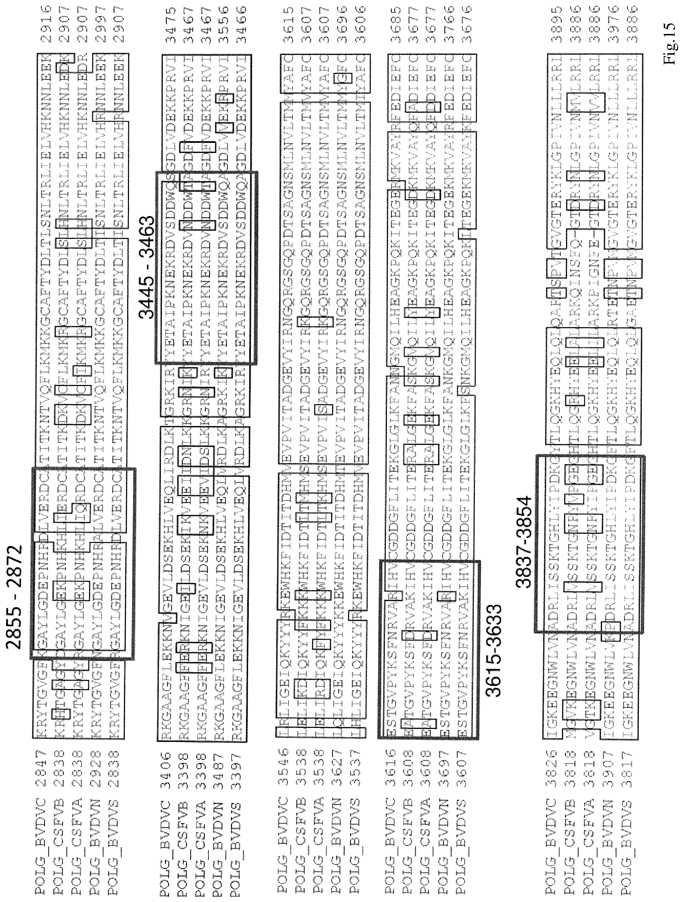

Fig. 15 shows the common/similar sequence information of the swine fever virus and the related viruses.
Fig. 16 shows a design application for other viruses.

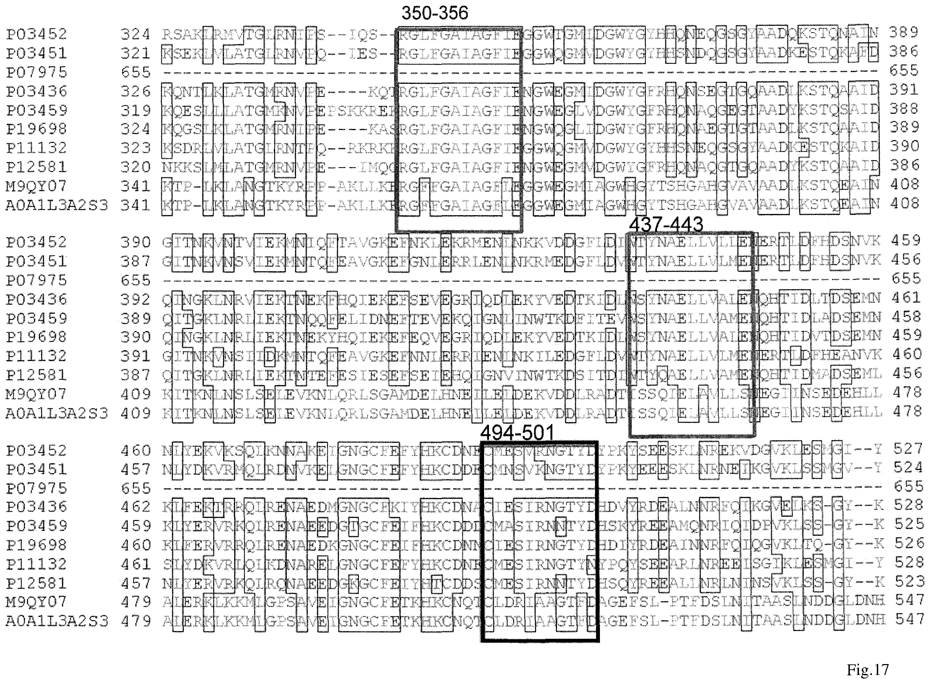

Fig. 17 shows the common/similar sequence information of influenza virus and related viruses.

### DESCRIPTION OF EMBODIMENTS

Next, embodiments of the present invention will be described, but the present invention is not limited to these embodiments.

An immunostimulant of the present invention is utilized as part of a cosmetic, a quasi-pharmaceutical product, a food, a feed additive in the case of livestock, an environmental cleaning preparation, and the like for each of nasal cavity mucous membrane, airway mucous membrane, and intestinal mucous membrane tissue. Even if a vaccine that induces IgG is developed like a conventional vaccine, it is a methodology for preparing for a case where the antibody has a weak point such that the antibody titer does not last for one year or more like an antibody against seasonal influenza. An object of the present preparation is to induce a highly crossed or diverse IgA antibody that relatively widely recognizes even a virus that is likely to be mutated, such as an RNA virus, if there is a certain similarity, by periodic use. In addition to a synergistic effect of a nasal vaccine, a respiratory vaccine, and an oral vaccine that have been independently developed so far, the synergistic effect is aimed by controlling an intestinal bacterial flora that affects immunostimulation.

Among viral surface proteins, a gene sequence used in the present invention is based on an epitope sequence common to or highly similar to a virulent virus and a virus that is closely related and is not virulent. However, unlike a conventional antigen epitope, the epitope is not necessarily limited to a hydrophilic region, is not based on a peptide of about 6 to 20 amino acids as the epitope, and is a fusion protein to which a candidate epitope is bound by a histidine tag described later or the like in order to make the sequence as an amino acid sequence as long as possible.

In addition, as a bacteria-derived protein, Heat shock protein 60 (HSP 60) and Heat shock protein 65 (HSP 65) are fused as adjuvant candidate proteins expected to be easily taken up by M cells in mucosal cells to construct a hybrid antigen.

Examples of HSP 60 that is an adjuvant candidate protein in a hybrid antigen include Bruccella aboritus, Clostridium difficle, Salmonella typhimurium, and Streptococcus suis. Examples of HSP 65 include HSP 65 derived from Mycobacterium leprae, which has been suggested to contribute to the activation of transairway immunity. Although both of them are bacteria-derived heat shock proteins positioned in BCL2, BCL3, or the like at a biosafety level, this is not the cause of pathogenicity, and it is an important point that the M cells are easily taken in. It is known that glycoprotein 2 (GP-2) and prion protein (PrP) are expressed in the M cells and play a role in a step of taking in an antigen molecule and presenting the antigen.

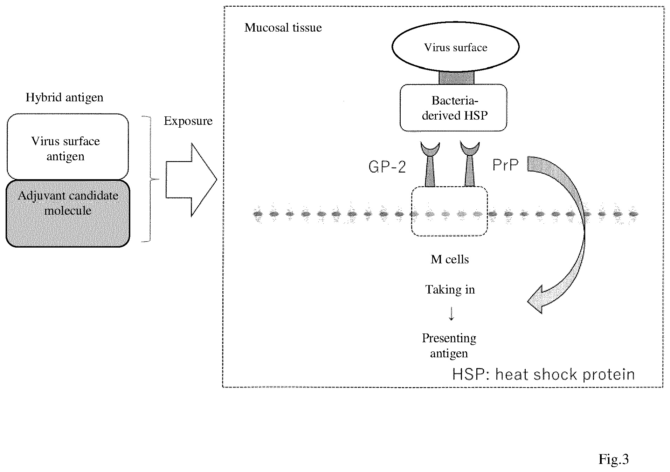

Therefore, in the present invention, it is expected that antigen presentation can be efficiently performed by utilizing the HSP described in paragraph [0029] so that the HSP is easily taken up by the M cells of the mucosal tissue in a form in which a surface antigen of the virus is reproduced as much as possible (Fig. 3).

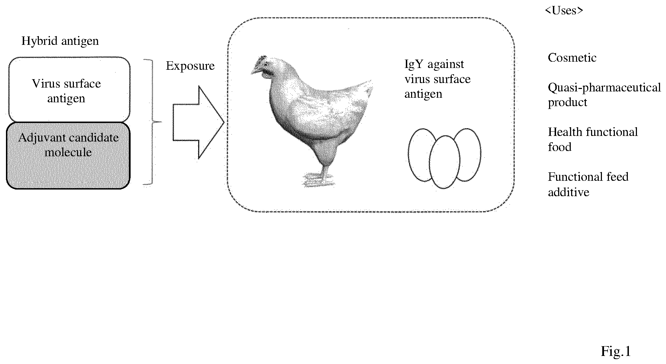

In a chicken (Non Patent Literature 7) having the M cells and having the M cells densely packed therein, by exposing the hybrid antigen, IgY derived from eggs and having high crossing-over property or high diversity can be acquired with high efficiency. By utilizing this diverse IgY, the immunostimulant is utilized as part of a cosmetic, a quasi-pharmaceutical product, a food, a feed additive in the case of livestock, an environmental cleaning preparation, and the like, whereby the immunostimulant can be expected to further expand the range of applications (Fig. 1).

In order to enhance the effect of the immunostimulant of the present invention by controlling the intestinal bacterial flora, bacterial groups of Bacillus hisashii (International Deposit Number BP-863) and Bacteroidetes phylum, which are known to enhance production ability of IgA, lactic acid bacteria, yeast, and the like are expected to be important.

### (Example 1)

A fusion protein in which an epitope of the amino acid sequence described in Table 1 is bound to HSP via histidine is designed. After a crude purified product is mixed with any spreader or liquid by utilizing the crude purified product so as to be expressed in a wheat cell-free system, silkworm cell-free system, yeast system, or plant (tomato, rice, etc.) capable of protein synthesis, and nasal, respiratory, or oral administration is performed. The production of the fusion protein in edible crops is promising because it can contribute to reduction in production cost. However, in the current law, the fusion protein is included in a category of genetically modified crops, and thus generally requires time for approval. However, unlike general application development of genetic recombination, the present technology is not introduced with a molecule that causing disturbance of an ecosystem, but is used for medical use, and thus it is expected to be the background of the era in which the present technology is rapidly approved. As for the hybrid antigen, a fusion protein by a long chain peptide in which some short chain epitopes are linked is particularly recommended as No. 3 in Table 1. As the epitope, a method is conceivable in which a peptide having a total of 50 amino acids or less is bound by an amino acid selected from histidine and the like described later by adding a region that is not necessarily suitable in the front and back. The reason why the present invention is not limited to the short-chain epitope is to make immune tolerance by the short-chain epitope less likely to occur; however, this point is not the development target of the present invention, and is based on a known report.

### [Table 1]

**Table 1. Hybrid antigen candidate**

| Hybrid antigen No. | Epitope | Binding site | HSP |
|---|---|---|---|
| 1 | GAGICASYQTQT | (Histidine) n | HSP 65 derived from Mycobacterium leprae |
| 2 | GAGICASYHTVS | | HSP 60 derived from Streptococcus suis |
| 3 | GAGICASYQTQT and GAGICASYHTVS | | HSP 65 derived from Mycobacterium leprae |

### Epitope references: https://www.nature.com/articles/s41591-020-0820-9

The epitope sequence in Table 1 is a common sequence of a spike protein of the batacoronavirus of accession codes MN 908947, MN 996532, AY 278741, KY 417146, and MK 211376 in the NCBI gene bank. The sequence is also common to SARS-cov having infectivity to humans and SARS-cov-2, and is characterized by being a region that does not affect the infectivity.

### (Example 2)

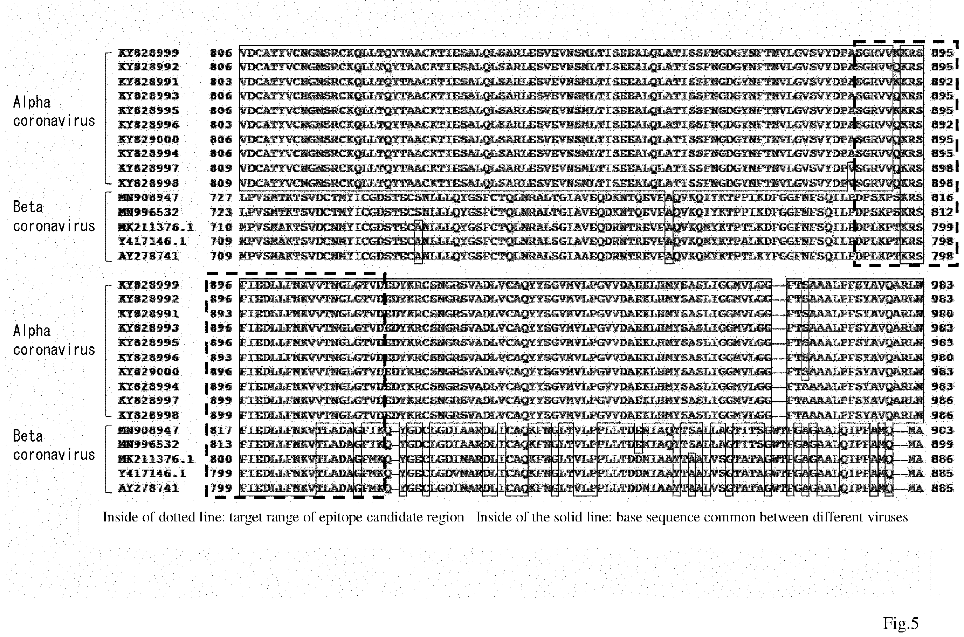

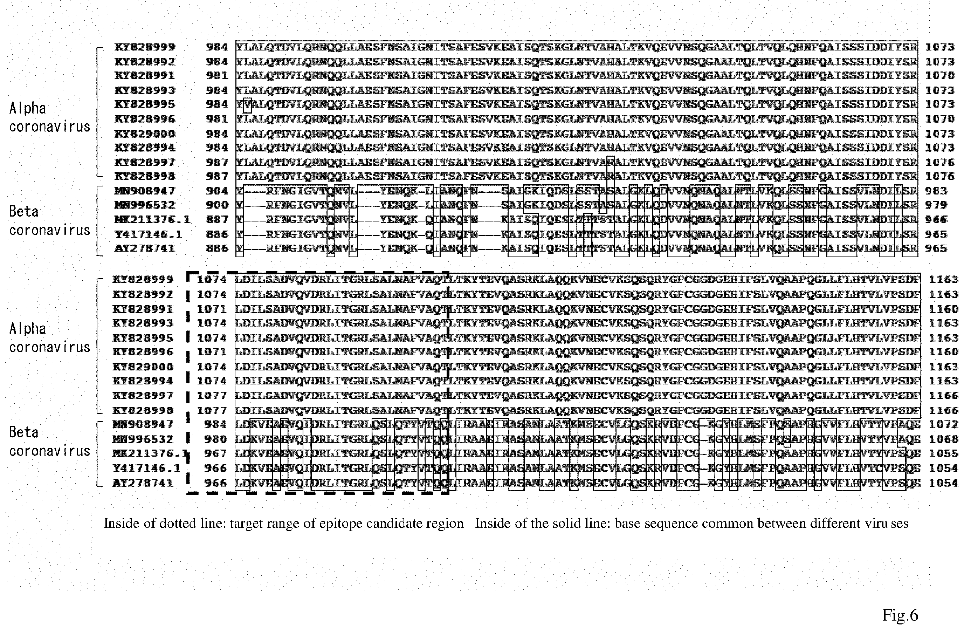

Comparative genome analysis was performed on the alphacoronavirus and the batacoronavirus for the gene sequence of the spike protein (Fig. 5 and Fig. 6). The range of the solid line surrounds a base sequence common between different viruses. As a result, a sequence region in the range of the dotted frame in Fig. 5 was a part of the spike protein of coronavirus, and was common or similar between both viruses. Also, the sequence region in the range of the dotted frame in Fig. 6 was a part of the spike protein of coronavirus, and was common or similar between both alpha and beta viruses.

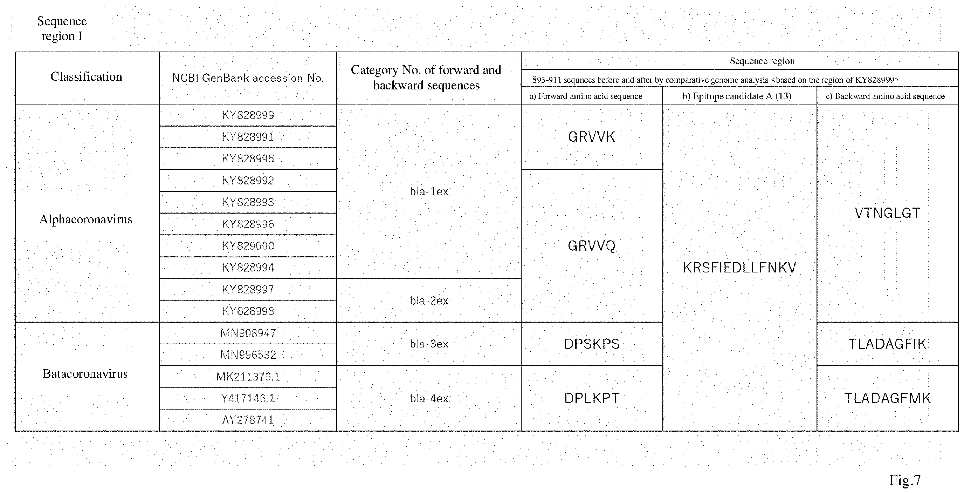

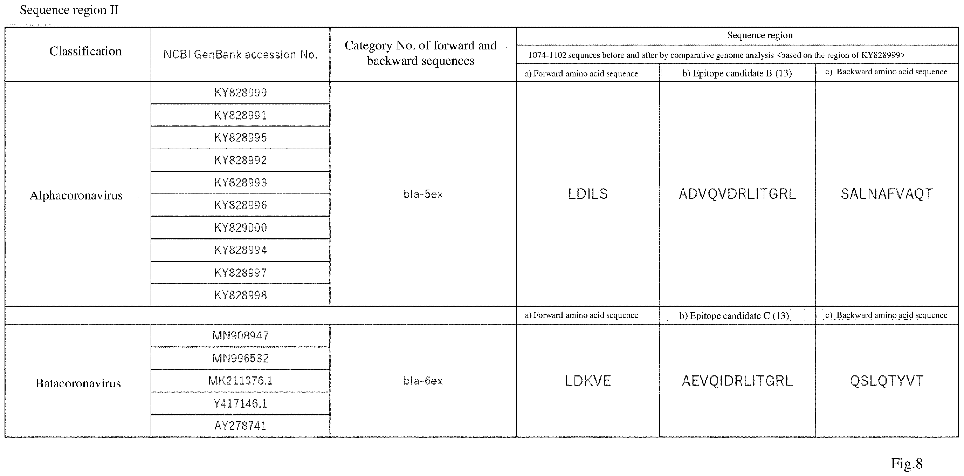

These sequences are as described in Fig. 7 and Fig. 8. In each case, the alphacoronavirus is based on a sequence published as a porcine epidemic diarrhea virus (PED; porcine epidemic diarrhea) (document name: Virus Genes (2013) 54: 215-224. https://www.ncbi.nlm.nih.gov/pmc/articles/PMC7088687/pdf/11262_2017_Article_152 8.pdf). Accession no. in each case is as described in the figure.

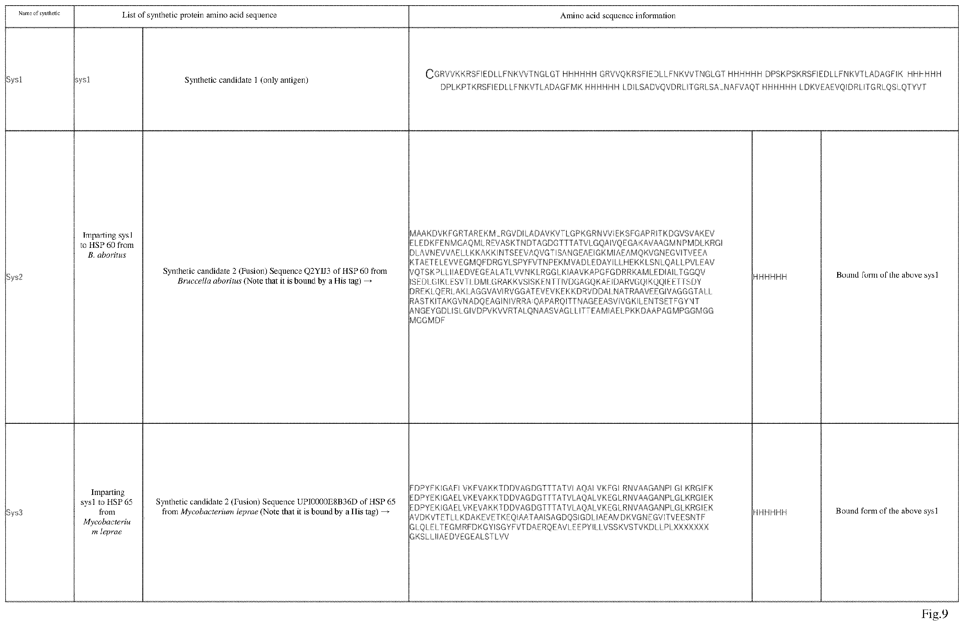

Thus, using these sequences as epitopes, a fusion protein bound to the downstream of the HSP of Fig. 9 via histidine is designed, and a hybrid antigen protein is synthesized so as to be expressed in a wheat cell-free system, a silkworm cell-free system, a yeast system, or a plant capable of protein synthesis. As an alternative to the histidine tag, the amino acid having a moderate hydropathic level such as HQ tag (HQHQHQ), HN tag (HNHNHNHNHNHN), HAT tag (KDHLIHNVHKEEHAHAHNK), cystetag (CCPGCC), tag of cysteine itself (C or CCCCCC), and methionine (M) is assumed, depending on the material for spreading the symbiotic preparation, asparagine (N), glutamic acid (E), and threonine (T) are assumed as the hydrophilic amino acid, and alanine (A), tyrosine (Y), and the like are assumed as the hydrophobic amino acid.

### (Example 3)

### Method:

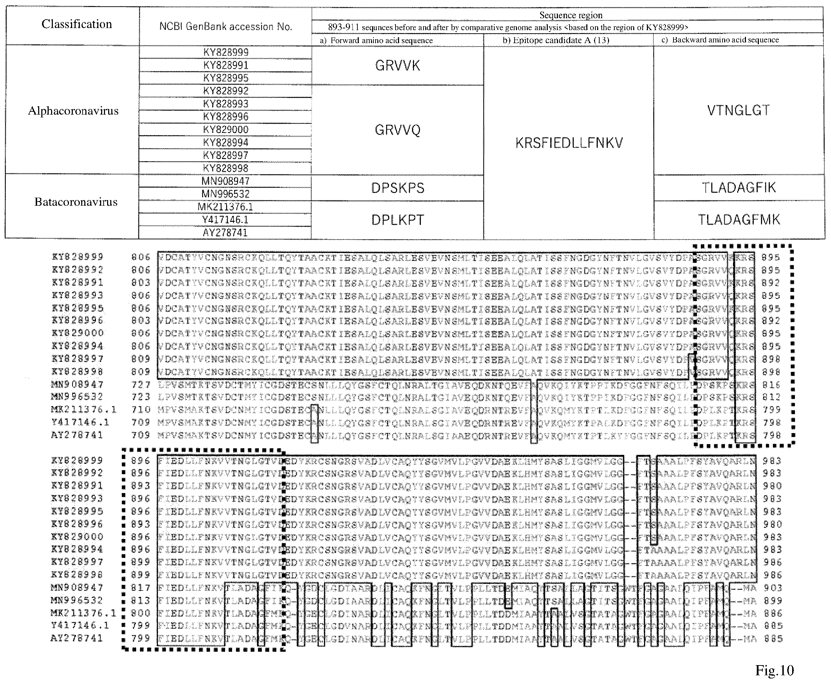

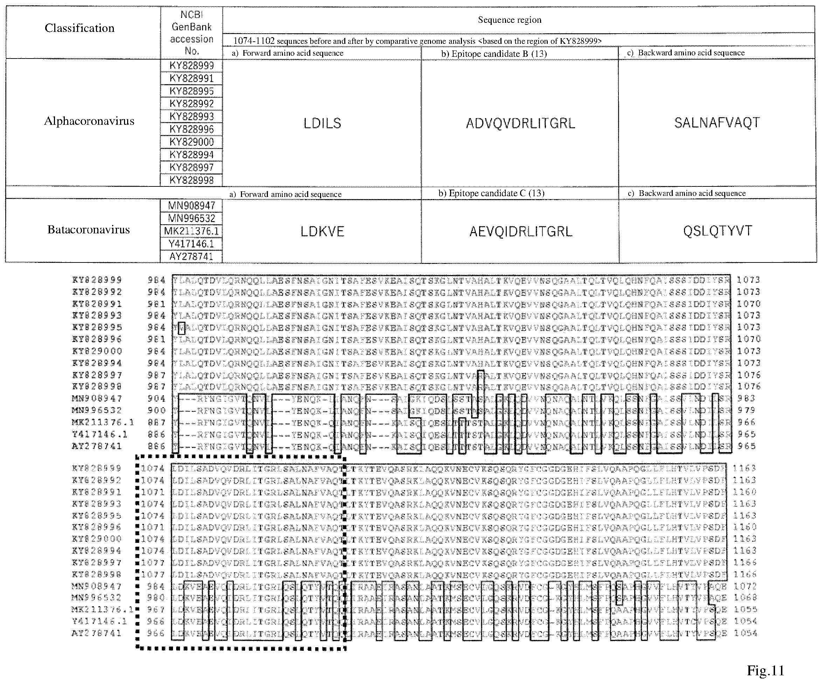

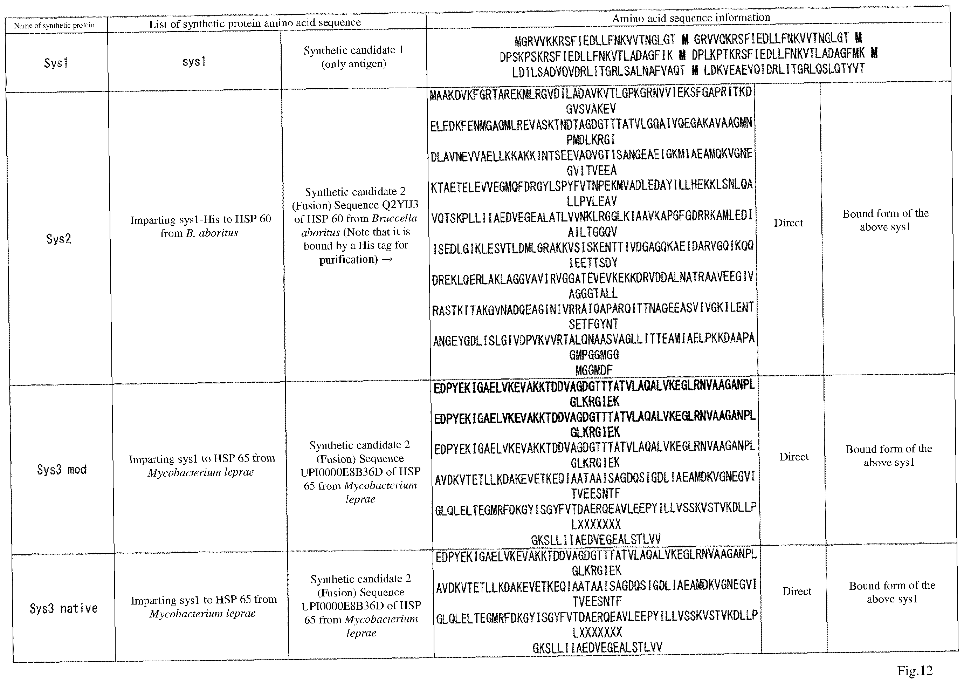

A peptide sequence to which about five amino acids before and after the epitope candidate described in Fig. 10 and Fig. 11 were imparted was designed using common/similar sequence regions of alphacoronavirus and betacoronavirus as targets. Based on these, they were further bound, and Sys1 in Fig. 12 was designed as a long chain peptide as an epitope candidate. As a protein to be fused with the Sys1, Heat shock protein and Heat shock protein modified with some amino acids were bound to prepare four kinds of fusion proteins. As protein synthesis, a cell-free synthesis system was used. Specifically, protein synthesis/purification at a 6 mL scale was performed using a fully automated protein synthesizer/purifier, Protemist DTII. In the purification, a synthetic protein was bound to a nickel resin, and the resin was washed with a washing buffer, and then eluted with imidazole (imidazole concentration of washing buffer: 50 mM). For quantification of a target protein, a part of the synthetic protein (total fraction) was centrifuged at 21,600 x g for 10 minutes at 4°C, and divided into a supernatant (soluble) fraction and a precipitated (insoluble) fraction, each fraction and purified protein were subjected to SDS-PAGE, then proteins were detected by CBB staining, and simultaneously migrated BSA was used as a standard protein to calculate the quantification (WEPRO used: 7240H).

### Result:

As described in Table 2, the solubility was low and 10% or less with only the long chain peptide; however, the solubility of the HSP and the fusion protein fused with the amino acid-modified HSP remarkably increased on average.

### [Table 2]

**Table 2. Evaluation of influence on solubilization rate of fusion protein**

| Long chain peptide antigen epitope candidate sequence | Long chain peptide antigen epitope candidate + HSP fusion protein (3 types) |
|---|---|
| < 10% | 82.0 ± 19.3% |

| | |
|---|---|
| Buffer composition of purified protein: 20 mM Na-Phosphate pH7.5, 300 mM NaCl, 500 mM Imidazole | |

Based on these results, since the HSP and the fusion protein of the amino acid-modified HSP and the long chain peptide epitope had increased solubility, the possibility of application to a vaccine or the like fused with an adjuvant was expected.

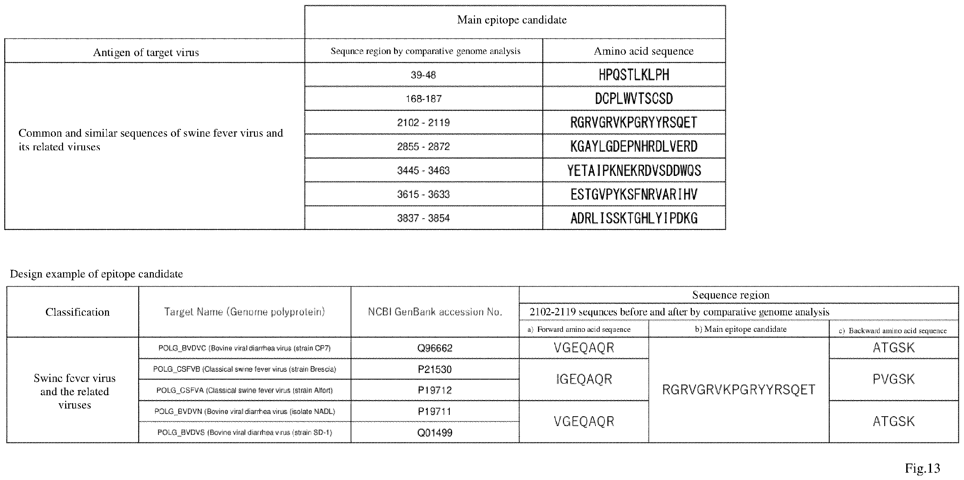

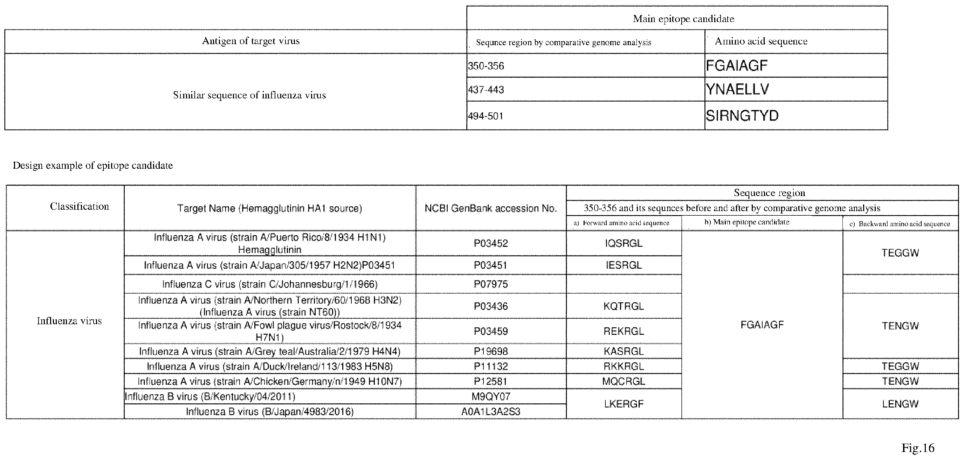

Based on the above results, a long chain peptide was designed for the common sequence of influenza virus, swine fever virus (pestivirus) and its related viruses, and similar sequences (Figs. 10 to 13, Fig. 16). In the future, by synthesis of a fusion protein of these long chain peptides and HSP, synthesis of a vaccine of a type that stimulates cross immunity that expects a wide range of immune effects that can withstand viral mutations is expected.

By promoting the binding between the HSP and the M cells in the mucosal system, it is expected that a new vaccine strategy can be developed by stimulating the mucosal immune system orally and via the respiratory tract.

A gene expression test in tomato has already been performed, and the possibility of synthesis of the fusion protein in crops is suggested. If the gene is applied to a probiotic or a microorganism (lactic acid bacteria, yeasts, Bacillus subtilis, and the like) effective as a resident bacterium of the skin, it can also be expected that the resident bacterium constantly produces the fusion protein to activate a cross immune system. It is expected that legal problems will be avoided so that such next-generation research can be conducted.

As a result, it is possible to give a host a spike antigen stimulus having high diversity in common between the alphacoronavirus and the batacoronavirus, and various immune responses are possible; therefore, in this case, in particular, it is expected to induce resistance to unknown alphacoronavirus and batacoronavirus. The epitope candidate sequence region (sequences shown in Fig. 5 and Fig. 6) is expected as a base sequence that can be used as an invasive vaccine antigen including a DNA vaccine or an RNA vaccine in order to realize immune induction in common to at least the alphacoronavirus and the betacoronavirus.

The method is not limited to the alphacoronavirus and the betacoronavirus in particular, and is a method having a possibility for future unknown infectious diseases such as Arterivirus genus in Arteriviridae family involved in porcine reproductive and respiratory syndrome (PRRS), Simian hemorrhagic fever (Simian hemorrhagic fever virus, Simian haemorrhagic fever virus; SHFV), and the like, or Influenza virus genus belonging to Orthomyxoviridae family and requiring various types. Naturally, the sequence will use a common sequence between closely related viruses of the viridae of interest, unlike the sequence of the present application. That is, similarly to the present method, by producing a hybrid antigen in which a plurality of common regions and similar regions such as the surface antigen of a pathogenic microorganism closely related to a target pathogen are fused, it is expected that comprehensive biophylaxis function is enhanced and symbiosis with various pathogenic microorganisms becomes possible by administering these hybrid antigens.

In order to facilitate binding to a carrier protein, it is also recommended as one of the possibilities in Examples 1 and 2 that cysteine is added to an N-terminal to facilitate binding to a carrier protein using a sulfide group.

Incidentally, a method of binding a similar epitope as a plurality of kinds of long chain peptides rather than a short chain peptide by utilizing similarity of viruses or the like is advantageous. This is because the possibility of preventing the possibility of immune tolerance at the epitope by the short chain peptide is expected. In addition, it is expected that the possibility of antibody-dependent immune enhancement (ADE: Antibody-dependent enhancement) due to incomplete antigen presentation is avoided. Although it is known that a long chain peptide in which an epitope of a helper T cell and a CTL epitope are bound to each other has a high vaccine effect, it is expected that the vaccine effect is further enhanced by binding in combination with a fusion protein of the viral gene. In addition, by non-invasively stimulating the mucosal tissue by nasal, respiratory, or oral administration, and binding to the M cell of the mucosal cell using bacteria-derived heat shock protein 60 (HSP 60) or heat shock protein 65 (HSP 65) to activate the immune system, it is expected that an immunoglobulin having high crossing-over property or high diversity can be induced against a wide range of environmental microorganisms. If the ADE is induced in the form of such a fusion protein, it is expected that it can be utilized as a tool useful for elucidating a mechanism of action of the ADE whose mechanism is unclear.

The origin of the term "vaccine" is a synonym related to cowpox used in the above-mentioned study of Edward Jenner, and is derived from Vacca (cow) in Latin. Therefore, in the preparation of the present invention, although an original idea of the vaccine is inherited, the viewpoint of placing importance on symbiosis with a wide range of environmental microorganisms is added, and, as a preparation considering the ecosystem, the concept is proposed as Symbioccin from Ecoccin and Symbiosis which means symbiosis.

The epitope sequence and sequence information of the HSP in the fusion protein are expected to be replaced by the base sequence that further improves the cross immune system. In addition to preparing and using the fusion protein in advance, a method of constantly expressing the protein synthesis by gene introduction into an effective microorganism such as yeast and lactic acid bacteria, which may be always present in the skin, can be studied. In addition, as a method of utilizing cells on the host side instead of utilizing resident microorganisms, a method of introducing a gene itself into a lipid-soluble capsule and applying the lipid-soluble capsule into which the gene has been introduced to skin cells or the like, thereby allowing the cells on the host side to express the gene expression of the fusion protein, can also be considered. If these steps of effect verification clear legal problems and the like and clear safety problems by various basic experiments and clinical experiments, an effect of activating cross-immunity is expected for a wide range of pathogenic microorganisms.

### INDUSTRIAL APPLICABILITY

Since it is suggested that a fermented product containing thermophilic bacteria (NITE International Deposit Number: BP-863) and thermophilic complex bacteria (ATCC International Deposit Number: PTA-1773) used as probiotic candidates has a possibility of inducing the expression of interferon, which is a cytokine involved in protection against viral infection, after improving the intestinal flora (Journal of Bioscience and Bioengineering, 114(5): 500-505, 2012; the Gene Expression Omnibus (GEO) database (access ID: GSE37732)), a possibility of enhancing the activation action of the immune system by combination use with the HSP fusion protein described in the present specification is expected. Therefore, application development as a novel preparation mixed with the fusion protein is expected.

## Claims

1. An immunostimulatory preparation comprising a fusion protein in which heat shock protein (HSP) or the amino acid-modified HSP is bound to a peptide epitope sequence, and solubility is improved as compared with the condition of the peptide epitope alone.

2. The immunostimulatory preparation according to claim 1, further comprising a fusion protein in which a plurality of types of units including different base sequences are bound before and after the peptide epitope, and then the HSP or the amino acid-modified HSP is bound.

3. The immunostimulatory preparation according to claim 1 or 2, wherein after the HSP or the amino acid-modified HSP is taken up by M cells of mucous membranes in a nasal cavity, a respiratory tract, and an intestinal tract and the peptide epitope is presented as an antigen, and then cross immunity against a microorganism which is likely to be mutated is induced depending on the sequence of the peptide epitope and properties of different base sequences before and after the peptide epitope.

4. The immunostimulatory preparation according to claims 1 to 3, further comprising, as a unit including different base sequences before and after the peptide epitope, an epitope on a surface of a virus common to or similar to various strains of a coronavirus that is likely to be mutated and a sequence before and after the epitope, the sequence being at least an amino acid sequence of any one of SEQ ID NO: 28 common to an alphacoronavirus and a betacoronavirus, and SEQ ID NO: 29 or SEQ ID NO: 30 similar between the alphacoronavirus and the betacoronavirus.

5. The immunostimulatory preparation according to claims 1 to 3, wherein the microorganism which is likely to be mutated is a pestivirus, and the peptide epitope includes at least any one amino acid sequence of SEQ ID NOs: 31 to 37.

6. The immunostimulatory preparation according to claims 1 to 3, wherein the microorganism which is likely to be mutated is an influenza virus, and the peptide epitope includes at least any one amino acid sequence of SEQ ID NOs: 38 to 40.
